# EUROPEAN PATENT SPECIFICATION

(11) **EP 4 294 450 B1**
(45) Date of publication and mention of the grant of the patent: **04.02.2026**
(21) Application number: 22756826.8
(22) Date of filing: 16.02.2022
(51) Int. Cl.: A61K 9/51, A61K 47/69, C12N 15/88, A61K 31/713, A61K 48/00

(54) **METHODS FOR PREPARATION OF PLASMID DNA/LIPID PARTICLES WITH DEFINED SIZE FOR IN VITRO AND IN VIVO TRANSFECTION**
VERFAHREN ZUR HERSTELLUNG VON PLASMID-DNA/LIPID-PARTIKELN MIT DEFINIERTER GRÖSSE ZUR IN-VITRO- UND IN-VIVO-TRANSFEKTION
PROCÉDÉS DE PRÉPARATION DE PARTICULES LIPIDIQUES/ADN PLASMIDIQUE DE TAILLE DÉFINIE POUR LA TRANSFECTIONET IN VITRO ET IN VIVO

(30) Priority: 16.02.2021 US 202163149985 P
(43) Date of publication of application: 27.12.2023
(73) Proprietor: The Johns Hopkins University, Baltimore, Maryland 21218 (US)
(72) Inventor: MAO, Hai-Quan, Baltimore, MD 21218 (US); ZHU, Yining, Baltimore, MD 21218 (US); HU, Yizong, Baltimore, MD 21218 (US)
(74) Representative: Murgitroyd & Company
(86) International application number: PCT/US2022/016580
(87) International publication number: WO 2022/177977

(56) References cited:
- WO-A1-2017/218704
- WO-A1-2021/016430
- WO-A2-2020/104970
- RU-A- 2014 122 432
- PEZZOLI D. ET AL.: "Size matters for in vitro gene delivery: investigating the relationships among complexation protocol, transfection medium, size and sedimentation", vol. 7, no. 1, 8 March 2017 (2017-03-08), US, XP093215287, ISSN: 2045-2322, Retrieved from the Internet <URL:https://www.nature.com/articles/srep44134.pdf> DOI: 10.1038/srep44134
- HAN X. ET AL.: "The heterogeneous nature of polyethylenimine-DNA complex formation affects transient gene expression", vol. 60, no. 1-3, 1 July 2009 (2009-07-01), Dordrecht, pages 63 - 75, XP093215293, ISSN: 0920-9069, Retrieved from the Internet <URL:http://link.springer.com/article/10.1007/s10616-009-9215-y/fulltext.html> DOI: 10.1007/s10616-009-9215-y
- XIE Q. ET AL.: "PEI/DNA formation affects transient gene expression in suspension Chinese hamster ovary cells via a one-step transfection process", vol. 65, no. 2, 11 July 2012 (2012-07-11), Dordrecht, pages 263 - 271, XP093215298, ISSN: 0920-9069, Retrieved from the Internet <URL:http://link.springer.com/article/10.1007/s10616-012-9483-9/fulltext.html> DOI: 10.1007/s10616-012-9483-9
- G�MEZ-AGUADO I. ET AL.: "Nucleic Acid Delivery by Solid Lipid Nanoparticles Containing Switchable Lipids: Plasmid DNA vs. Messenger RNA", MOLECULES, vol. 25, no. 24, 18 December 2020 (2020-12-18), CH, pages 5995, XP093214913, ISSN: 1420-3049, DOI: 10.3390/molecules25245995
- KORABECNA M., ZINKOVA A., BRYNYCHOVA I., CHYLIKOVA B., PRIKRYL P., SEDOVA L., NEUZIL P., SEDA O.: "Cell-free DNA in plasma as an essential immune system regulator", SCIENTIFIC REPORTS - NATURE RESEARCH, vol. 10, no. 1, 1 December 2020 (2020-12-01), XP055965799, DOI: 10.1038/s41598-020-74288-2

## Description

### BACKGROUND

Lipid nanoparticles (LNPs) have been widely used to deliver nucleic acids for gene therapy. Factors, such as lipid and nucleic acid composition, nitrogen/phosphate (N/P) ratio and biodegradability of LNPs have been widely explored for boosting *in vitro* and *in vivo* transfection efficiency. Current methods known in the art for producing viral vectors, however, have been limited by poor reproducibility, inconsistent yield, and less than optimal *in vitro* and *in vivo* transfection efficiency. For example, discrete sub-100 nm nanoparticles have been successfully generated in a lyophilized form for systemic delivery applications *in vivo.* Hu et al., 2019. These small nanoparticles, however, are sub-optimal for *in vitro* transfection in viral vector production cell lines (i.e., HEK293T or HEK293F cells) and other cell types. All three LNP-based formulations used to deliver siRNA and COVID-19 mRNA vaccines and some lipid NP-based products in clinical trials for targeting liver (i.e., MTL-CEBPA for inhibiting liver cancer, ND-L02-s0201/BMS-986263 for liver fibrosis) all have particles in the size range of 40 nm to 200 nm. There have been no reports on how LNPs in the range of 200 nm to 1200 nm influence the transfection efficiency both *in vitro* and *in vivo.* This dearth of information is largely due to the lack of reliable methods or processes to allow the preparation of lipid nanoparticles with a tunable size within this range.

### SUMMARY

In some aspects, the presently disclosed subject matter provides a method for preparing a plurality of nucleic acid/lipid particles having an average particle size ranging from 210 to 1200 nm and a polydispersity index from 0.05 to 0.5, the method comprising:
(a) preparing or providing a first solution comprising a plurality of nucleic acid/lipid nanoparticles having a first particle size ranging from 20 to 200 nm;
(b) reducing a polarity of the first solution from a dielectric constant of about 80 to about 45 to 60 to induce particle-size growth of the plurality of nucleic acid/lipid nanoparticles having a first particle size to form a second solution comprising a plurality of nucleic acid/lipid nanoparticles having a second particle size, wherein the second particle size is in the range of 210 nm to 1200 nm;
(c) reversing the polarity of the second solution with a dielectric constant of about 65 to 80 to halt growth of the plurality of nucleic acid/lipid nanoparticles having a second particle size at a predetermined particle size, to form a plurality of nucleic acid/lipid particles having a defined particle size ranging from 210 to 1200 nm and a polydispersity index from 0.1 to 0.3.

In certain aspects, the first particle size has a range between about 40 nm to about 120 nm. In particular aspects, the first particle size is about 60 nm.

In certain aspects, the second particle size has a range from about 210 nm to about 1,200 nm. In particular aspects, the second particle size is selected from the group consisting of about 210, 300, 400, 500, 600, 700, 800, 900, 1000, 1100, and 1200 nm.

In certain aspects, the first solution further comprises one or more multivalent cations selected from the group consisting of Ca²⁺, Mg²⁺, Zn²⁺, and Fe³⁺.

In certain aspects, the polarity of the first solution is reduced by adding a water-miscible organic solvent thereto. In particular aspects, the method comprises adding a varying concentration of the water-miscible organic solvent to the first solution to control the particle-size growth of the plurality of nucleic acid/lipid nanoparticles having a first particle size. In more particular aspects, the water-miscible organic solvent is selected from the group consisting of ethanol, methanol, butanol, isopropanol, dimethyl sulfoxide (DMSO), dimethylformamide (DMF), tetrahydrofuran (THF), acetone, and acetonitrile.

In certain aspects, the polarity of the second solution is reversed via dilution with H₂O.

In certain aspects, the first solution comprising a plurality of nucleic acid/lipid nanoparticles having a first particle size is prepared by a continuous flash nanocomplexation (FNC) technique. In other aspects, the first solution comprising a plurality of nucleic acid/lipid nanoparticles having a first particle size is prepared by a manual pipette mixing and vortexing method. In particular aspects, the method comprises an input nucleic acid concentration of about 100 µg/mL.

In certain aspects, the nucleic acid is selected from the group consisting of an antisense oligonucleotide, cDNA, genomic DNA, guide RNA, plasmid DNA, vector DNA, mRNA, miRNA, piRNA, shRNA, and siRNA. In particular aspects, the nucleic acid comprises plasmid DNA (pDNA) or a mixture of different species of plasmid DNA. In yet more particular aspects, the nucleic acid comprises plasma DNA.

In some aspects, the nucleic acid/lipid nanoparticle comprises one or more lipids selected from the group consisting of positively charged lipids, neutral lipids, negatively charged lipids, PEGylated lipids, and ionizable lipids.

In certain aspects, nucleic acid/lipid nanoparticle comprises a cationic lipid. In particular aspects, the cationic lipid is selected from the group consisting of N1-[2-((1S)-1-[(3-aminopropyl)amino]-4-[di(3-amino-propyl)amino]butylcarboxamido) ethyl]-3,4-di[oleyloxy]-benzamide, 1,2-di-O-octadecenyl-3-trimethylammonium propane (DOTMA), O-alkyl phosphatidylcholines, 1,2-dilauroyl-sn-glycero-3-ethylphosphocholine (12:0 EPD), 1,2-dimyristoyl-sn-glycero-3-ethylphosphocholine (14:0 EPC), 1,2-dipalmitoyl-sn-glycero-3-ethylphosphocholine (16:0 EPC), 1,2-distearoyl-sn-glycero-3-ethylphosphocholine (18:0 EPC), 1,2-dioleoyl-sn-glycero-3-ethylphosphocholine (18:1 EPC), 1-palmitoyl-2-oleoyl-sn-glycero-3-ethylphosphocholine (16:0-18:1 EPC), and 1,2-dimyristoleoyl-sn-glycero-3-ethylphosphocholine (14:1 EPC), dimethyldioctadecylammonium (DDAB), N-(4-carboxybenzyl)-N,N-dimethyl-2,3-bis(oleoyloxy)propan-1-aminium (DOBAQ), 1,2-distearoyl-3-dimethylammonium-propane (18:0 DAP), 1,2-dipalmitoyl-3-dimethylammonium-propane (16:0 DAP), 1,2-dimyristoyl-3-dimethylammonium-propane (14:0 DAP), 1,2-dioleoyl-3-dimethylammonium-propane (DODAP) (18:1 DAP), 1,2-dimyristoyl-3-trimethylammonium-propane (14:0 TAP), 1,2-dipalmitoyl-3-trimethylammonium-propane (16:0 TAP), 1,2-stearoyl-3-trimethylammonium-propane (18:0 TAP), 1,2-dioleoyl-3-trimethylammonium-propane (18:1 TAP (DOTAP)), dioleoylphosphatidylethanolamine (DOPE), 3β-[N-(N',N'-dimethylaminoethane)-carbamoyl]cholesterol hydrochloride (DC-Cholesterol·HCl), DC-cholesterol, N4-Cholesteryl-Spermine (GL67), 1,2-dioleyloxy-3-dimethylaminopropane (DODMA), dimyristoyltrimethylammonium propane (DMTAP), 2,3,-dioleyloxy-N-[2(sperminecarboxamido)ethyl]-N,N-dimethyl-1-propane trifluoroacetate (DOSPA), N,N-dioleyl-N,N-dimethylammonium chloride (DODAC), 1,2-Dioleoylcarbamyl-3-Dimethylammonium-propane (DOCDAP), 1,2-Dilineoyl-3-Dimethylammonium-propane (DLINDAP), dilauryl(C_{12:0}) trimethyl ammonium propane (DLTAP), dioctadecylamidoglycyl spermine (DOGS), DC-Choi, 1,2-Dimyristyloxypropyl-3-dimethyl-hydroxyethyl ammonium bromide (DMRIE), 3-dimethylamino-2-(Cholest-5-en-3-beta-oxybutan-4-oxy)-1-(cis,cis-9,12-oc-tadecadienoxy)propane(CLinDMA), 2-[5'-(cholest-5-en-3[beta]-oxy)-3'-oxapentoxy)-3-dimethyl-1-(ci-s,cis-9',12'-octadecadienoxy) propane (CpLinDMA) and N,N-Dimethyl-3,4-dioleyloxybenzylamine (DMOBA), and 1,2-N,N'-Dioleylcarbamyl-3-dimethylaminopropane (DOcarbDAP), and combinations and pharmaceutically acceptable salts thereof.

In particular aspects, the cationic lipid is DOTAP.

### BRIEF DESCRIPTION OF THE FIGURES

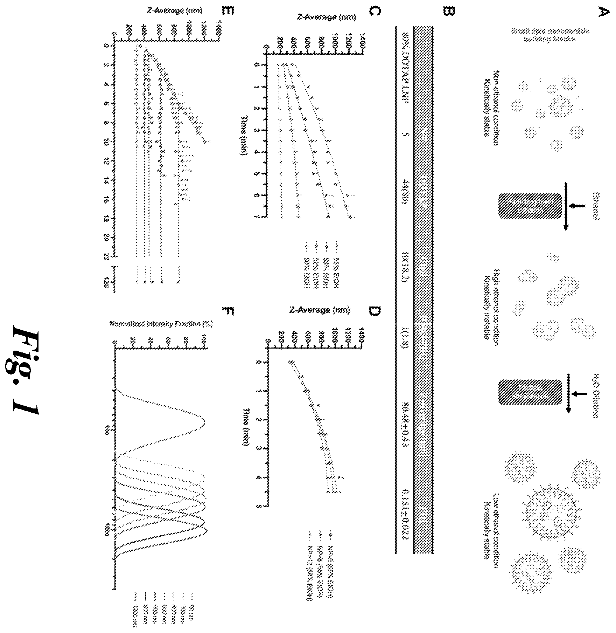

FIG. 1A, FIG. 1B, FIG. 1C, FIG. 1D, FIG. 1E, and FIG. 1F exemplify the presently disclosed method of size control for pDNA/lipid particles having a size ranging from about 60 nm to about 1200 nm through control of growth kinetics. FIG. 1A is a schematic of the proposed synthesis process. FIG. 1B shows formulation details and characterization of the designed lipid nanoparticles (LNP) used as small lipid nanoparticle building blocks. FIG. 1C demonstrates predictable size growth induced under different polarity of solvent (i.e., concentrations of ethanol). Error bars show s.e.m. derived from 3 independent experiments. FIG. 1D demonstrates predictable size growth of LNPs at different NP ratio induced by different concentrations of ethanol. FIG. 1E shows that particle size growth was halted by 1 to 1 dilution with H₂O at different time points along the growth curve with 55% ethanol. FIG. 1F shows the z-average diameter distributions measured by DLS of a series of stable particles with distinct sizes. Error bars show s.e.m. derived from 3 independent experiments for FIG. 1C, FIG. 1D and FIG. 1E;

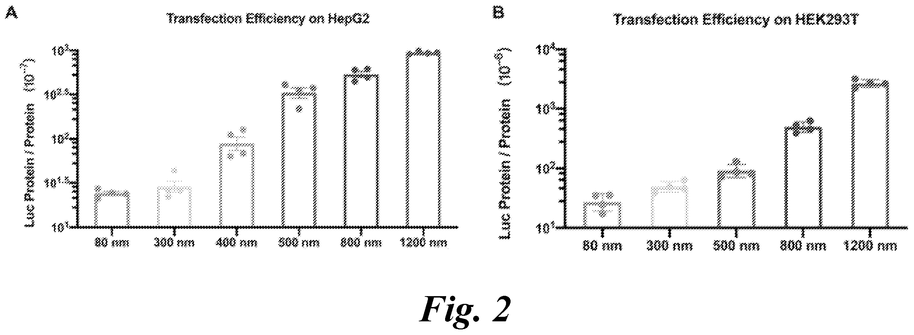

FIG. 2A and FIG. 2B show *in vitro* transfection efficiencies of stable particles with controlled size ranging from about 60 nm to about 1200 nm. The efficiency of transgene expression of luciferase as a reporter for Hek293T cells (FIG. 2A) or HepG2 cells (FIG. 2B). (n=4); and

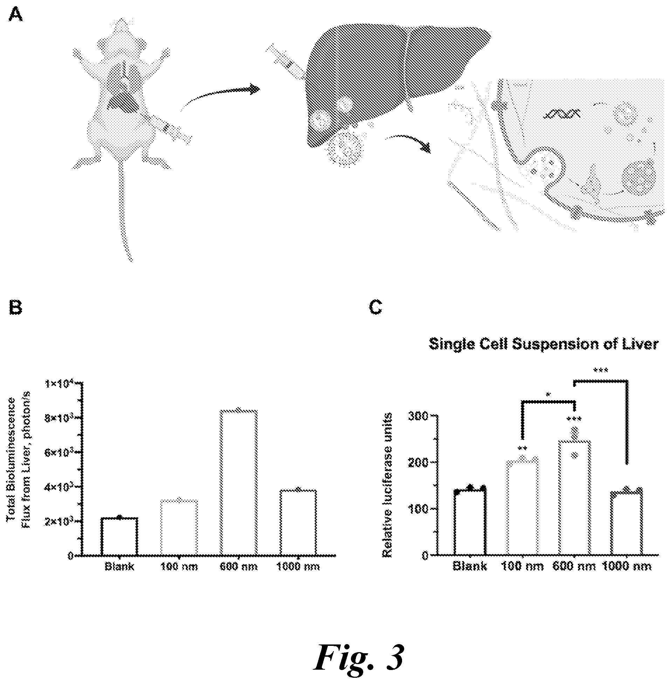

FIG. 3A, FIG. 3B, and FIG. 3C show *in vivo* local transfection efficiencies of stable pDNA/lipid particles with controlled size via intrahepatic injection. FIG. 3A is a representative scheme of intrahepatic injection; FIG. 3B shows the *in vivo* transfection efficiency in the liver in healthy BALB/c mice at 48-h post-intrahepatic injection of nanoparticles at 3 µg DNA per mouse; and FIG. 3C is the single cell suspension was further made from the collected liver tissue, and its luciferase activity was measured.

### DETAILED DESCRIPTION

The presently disclosed subject matter provides methods for preparing nucleic acid/lipid particles of an optimum particle size for efficient transfection of cells *in vitro* and *in vivo* local transfection are provided. The method is based on kinetic control of the nucleic acid/lipid nanoparticle assembly, including control of particle aggregation and growth arrest, to prepare shelf-stable particles with defined sizes between about 50 nm and 1200 nm. The size-dependent characteristics of the nucleic acid/lipid particle-mediated transfection in different cell lines for the size range between 50 nm and 1200 nm also is provided.

The presently disclosed subject matter represents the first systematic investigation of the size-dependent transfection for the size range greater than 200 nm and up to 1.2 µm. To date, DNA/lipid particles within this size range have not been well studied due to the limited interest for *in vivo* applications. To better understand the size effect of the DNA/lipid particles, the presently disclosed subject matter demonstrates the predictable nature of DNA/lipid particle aggregation and size growth kinetics. The presently disclosed method provides a scalable method for producing DNA/lipid particles with a higher degree of control of size within 210 and 1200 nm; furthermore, this method is highly versatile and can be tailored to produce DNA/lipid particles with various compositions.

The presently disclosed DNA/lipid particles yield superior and reproducible transfection activity and shelf stability and can be used as an off-the-shelf product. This DNA/lipid particle formulation can drastically simplify and streamline cell transfection processes, including the viral manufacturing process. In addition, this method improves the cell and viral vector production quality and consistency due to better quality control of DNA/lipid particles as the transfection agent. It is anticipated that the presently disclosed DNA/lipid particles will find a wide range of applications in process engineering for production of viral vectors at various scales. Other potential utility includes *ex vivo* transfection of cells for regenerative therapy and immunotherapy and *in vivo* gene therapy.

The presently disclosed size-defined nanoparticles potentially can find utility for *in vitro* and *ex vivo* localized transfection of cells for viral production or cell therapy, and *in vivo* localized transfection, including transfection of hepatocytes, keratinocytes, adult stem and progenitor cells, pluripotent stem cells, T cells, NK cells, and tumor cells.

**In** some embodiments, the presently disclosed subject matter provides a method for preparing a plurality of nucleic acid/lipid particles having an average particle size ranging from 210 to 1200 nm and a polydispersity index from 0.05 to 0.5, the method comprising:
(a) preparing or providing a first solution comprising a plurality of nucleic acid/lipid nanoparticles having a first particle size ranging from 20 to 200 nm;
(b) reducing a polarity of the first solution from a dielectric constant of 80 to about 45 to 60 to induce particle-size growth of the plurality of nucleic acid/lipid nanoparticles having a first particle size to form a second solution comprising a plurality of nucleic acid/lipid nanoparticles having a second particle size, wherein the second particle size is in the range of 210 nm to 1200 nm;
(c) reversing the polarity of the second solution with a dielectric constant of about 65 to 80 to halt growth of the plurality of nucleic acid/lipid nanoparticles having a second particle size at a predetermined particle size, to form a plurality of nucleic acid/lipid particles having a defined particle size ranging from 210 to 1200 nm and a polydispersity index from 0.1 to 0.3.

In certain embodiments, the first particle size has a range between about 40 nm to about 120 nm, including about 40, 45, 50, 55, 60, 65, 70, 75, 80, 85, 90, 95, 100, 105, 110, 115, and 120 nm. In more particular embodiments, the first particle size is about 60 nm.

In certain embodiments, the second particle size has a range from about 210 nm to about 1,200 nm. In particular embodiments, the second particle size is selected from the group consisting of about 210, 300, 400, 500, 600, 700, 800, 900, 1000, 1100, and 1200 nm.

In certain embodiments, the first solution further comprises one or more multivalent cations selected from the group consisting of Ca²⁺, Mg²⁺, Zn²⁺, and Fe³⁺.

In certain embodiments, the polarity of the first solution is reduced by adding a water-miscible organic solvent thereto. In particular embodiments, the method comprises adding a varying concentration of the water-miscible organic solvent to the first solution to control the particle-size growth of the plurality of nucleic acid/lipid nanoparticles having a first particle size. In more particular embodiments, the water-miscible organic solvent is selected from the group consisting of ethanol, methanol, butanol, isopropanol, dimethyl sulfoxide (DMSO), dimethylformamide (DMF), tetrahydrofuran (THF), acetone, and acetonitrile.

In certain embodiments, the polarity of the second solution is reversed via dilution with H₂O.

In certain embodiments, the first solution comprising a plurality of nucleic acid/lipid nanoparticles having a first particle size is prepared by a continuous flash nanocomplexation (FNC) technique. In other embodiments, the first solution comprising a plurality of nucleic acid/lipid nanoparticles having a first particle size is prepared by a manual pipette mixing and vortexing method. In particular embodiments, the method comprises an input nucleic acid concentration of about 100 µg/mL.

In certain embodiments, the nucleic acid is selected from the group consisting of an antisense oligonucleotide, cDNA, genomic DNA, guide RNA, plasmid DNA, vector DNA, mRNA, miRNA, piRNA, shRNA, and siRNA. In particular embodiments, the nucleic acid comprises plasmid DNA (pDNA) or a mixture of different species of plasmid DNA. In yet more particular embodiments, the nucleic acid comprises plasma DNA.

In certain embodiments, the presently disclosed nucleic acid/lipid particles are prepared with one or more lipids with different charge characteristics. In some embodiments, the one or more lipids are selected from positively charged lipids, neutral lipids, negatively charged lipids, PEGylated lipids, and ionizable lipids.

In certain embodiments, nucleic acid/lipid nanoparticle comprises a cationic lipid.

Cationic lipids have the ability to form aggregate complexes with anionic nucleic acids, such as DNA or RNA. These aggregated liposomal structures have a positive surface charge at physiologic pH. The positively-charged surface mediates the interaction of the anionic nucleic acid and the cell membrane and allows the introduction of the anionic nucleic acid to the cell due to the inability to distinguish the liposome from the cell membrane. Advantages of cationic lipid-mediated transfection include high efficiency, ability to transfect a wide range of cell types, reproducibility, low toxicity, and simplicity.

Suitable cationic lipids include, but are not limited to a multivalent cationic lipid, such as N1-[2-((1S)-1-[(3-aminopropyl)amino]-4-[di(3-aminopropyl)amino]butylcarboxamido)ethyl]-3,4-di[oleyloxy]-benzamide, 1,2-di-O-octadecenyl-3-trimethylammonium propane (DOTMA), O-alkyl phosphatidylcholines, such as ethylphosphocholines (EPCs), including 1,2-dilauroyl-sn-glycero-3-ethylphosphocholine (12:0 EPD), 1,2-dimyristoyl-sn-glycero-3-ethylphosphocholine (14:0 EPC), 1,2-dipalmitoyl-sn-glycero-3-ethylphosphocholine (16:0 EPC), 1,2-distearoyl-sn-glycero-3-ethylphosphocholine (18:0 EPC), 1,2-dioleoyl-sn-glycero-3-ethylphosphocholine (18:1 EPC), 1-palmitoyl-2-oleoyl-sn-glycero-3-ethylphosphocholine (16:0-18:1 EPC), and 1,2-dimyristoleoyl-sn-glycero-3-ethylphosphocholine (14:1 EPC), dimethyldioctadecylammonium (DDAB), pH sensitive cationic lipids, such as N-(4-carboxybenzyl)-N,N-dimethyl-2,3-bis(oleoyloxy)propan-1-aminium (DOBAQ), 1,2-distearoyl-3-dimethylammonium-propane (18:0 DAP), 1,2-dipalmitoyl-3-dimethylammonium-propane (16:0 DAP), 1,2-dimyristoyl-3-dimethylammonium-propane (14:0 DAP), 1,2-dioleoyl-3-dimethylammonium-propane (DODAP) (18:1 DAP), 1,2-dimyristoyl-3-trimethylammonium-propane (14:0 TAP), 1,2-dipalmitoyl-3-trimethylammonium-propane (16:0 TAP), 1,2-stearoyl-3-trimethylammonium-propane (18:0 TAP), 1,2-dioleoyl-3-trimethylammonium-propane (18:1 TAP (DOTAP)), dioleoylphosphatidylethanolamine (DOPE), 3β-[N-(N',N'-dimethylaminoethane)-carbamoyl]cholesterol hydrochloride (DC-Cholesterol·HCl), DC-cholesterol, N4-Cholesteryl-Spermine (GL67), 1,2-dioleyloxy-3-dimethylaminopropane (DODMA), dimyristoyltrimethylammonium propane (DMTAP), 2,3,-dioleyloxy-N-[2(sperminecarboxamido)ethyl]-N,N-dimethyl-1-propane trifluoroacetate (DOSPA), N,N-dioleyl-N,N-dimethylammonium chloride (DODAC), 1,2-Dioleoylcarbamyl-3-Dimethylammonium-propane (DOCDAP), 1,2-Dilineoyl-3-Dimethylammonium-propane (DLINDAP), dilauryl(C_{12:0}) trimethyl ammonium propane (DLTAP), dioctadecylamidoglycyl spermine (DOGS), DC-Choi, 1,2-Dimyristyloxypropyl-3-dimethyl-hydroxyethyl ammonium bromide (DMRIE), 3-dimethylamino-2-(Cholest-5-en-3-beta-oxybutan-4-oxy)-1-(cis,cis-9,12-oc- tadecadienoxy)propane (CLinDMA), 2-[5'-(cholest-5-en-3[beta]-oxy)-3'-oxapentoxy)-3-dimethyl-1-(ci- s,cis-9',12'-octadecadienoxy) propane (CpLinDMA) and N,N-Dimethyl-3,4-dioleyloxybenzylamine (DMOBA), and 1,2-N,N'-Dioleylcarbamyl-3-dimethylaminopropane (DOcarbDAP), and pharmaceutically acceptable salts thereof. In particular embodiments, the cationic lipid is DOTAP.

As used herein, the terms "salt" or "salts" refers to an acid addition of a compound of the invention. "Salts" include in particular "pharmaceutically acceptable salts". The term "pharmaceutically acceptable salts" refers to salts that retain the biological effectiveness and properties of the cationic lipid and, which typically are not biologically or otherwise undesirable. In many cases, the cationic lipid is capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002). Representative pharmaceutically acceptable salts include, but are not limited to, chloride, bromide, triflate (trifluoromethanesulfonic acid), methyl sulfate, hydrochloride, trifluoroacetate, and the like.

A plurality of nucleic acid/lipid nanoparticles prepared by the method disclosed herein, can be used in:
(a) *in vitro* transfection for viral vector production;
(b) *ex vivo* transfection for therapeutic cells and gene editing;
(c) *in vivo* transfection;
(d) *in vivo* local administration, such as intrahepatic injection, intradermal injection, intravitreal injection, intramuscular injection, intra-ear canal injection, and intratumor injection; and
(e) tissue-specific delivery, such as intranasal delivery, oral delivery, and sublingual delivery.

In *vitro* transfection for viral vector production comprises contacting one or more cells with the plurality of nucleic acid/lipid nanoparticles, e.g., by dosing the plurality of nucleic acid/lipid nanoparticles to a monolayer culture of the one or more cells or a suspension culture of the one or more cells.

Illustrative examples of cells suitable for transfection include, but are not limited to CHO cells, BHK cells, MDCK cells, C3H 10T1/2 cells, FLY cells, Psi-2 cells, BOSC 23 cells, PA317 cells, WEHI cells, COS cells, BSC 1 cells, BSC 40 cells, BMT 10 cells, VERO cells, W138 cells, MRC5 cells, A549 cells, HT1080 cells, 293 cells, B-50 cells, 3T3 cells, NIH3T3 cells, HepG2 cells, Saos-2 cells, Huh7 cells, HeLa cells, W163 cells, 211 cells, 211A cells, or derivatives thereof.

The one or more cells can comprise viral packaging HEK293 cells, HEK293S cells, HEK293T cells, HEK293F cells, HEK293FT cells, HEK293FTM cells, HEK293 SG cells, HEK293SGGD cells, HEK293H cells, HEK293E cells, HEK293MSR cells, or HEK293A, or HEK293T cells adapted for suspension culture.

The viral vector can be a retroviral vector. Illustrative examples of retroviral vectors include but are not limited to vectors derived from Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus.

The viral vector can be a lentiviral vector. Illustrative examples of lentiviral vectors include but are not limited to vectors derived from HIV (human immunodeficiency virus; including HIV type 1, and HIV type 2); visna-maedi virus (VMV) virus; the caprine arthritis-encephalitis virus (CAEV); equine infectious anemia virus (EIAV); feline immunodeficiency virus (FIV); bovine immune deficiency virus (BIV); and simian immunodeficiency virus (SIV).

Lentiviral vectors can be derived from HIV-1 or HIV-2.

A transfer plasmid can encode a lentiviral vector that comprises a left (5') lentiviral LTR, a Psi packaging sequence (Ψ+), a central polypurine tract/DNA flap (cPPT/FLAP), a rev response element (RRE), a promoter operably linked to a polynucleotide encoding a therapeutic transgene, and a right (3') lentiviral LTR. Lentiviral vectors may optionally comprise post-transcriptional regulatory elements including, but not limited to, polyadenylation sequences, insulators, a woodchuck hepatitis virus posttranscriptional regulatory element (WPRE), a hepatitis B virus (HPRE), and the like.

A transfer plasmid can encode a lentiviral vector that comprises a modified left (5') lentiviral LTR comprising a heterologous promoter, a Psi packaging sequence (Ψ+), a central polypurine tract/DNA flap (cPPT/FLAP), a rev response element (RRE), a promoter operably linked to a polynucleotide encoding a therapeutic transgene, and a modified (3') lentiviral LTR.

A transfer plasmid can encode a lentiviral vector that comprises a modified 5' LTR wherein the U3 region of the 5' LTR is replaced with a heterologous promoter to drive transcription of the viral genome during production of viral particles. Examples of heterologous promoters include, for example, viral simian virus 40 (SV40) (e.g., early or late), cytomegalovirus (CMV) (e.g., immediate early), Moloney murine leukemia virus (MoMLV), Rous sarcoma virus (RSV), and herpes simplex virus (HSV) (thymidine kinase) promoters.

A transfer plasmid can encode a lentiviral vector that comprises a modified self-inactivating (SIN) 3' LTR that renders the viral vector replication defective. SIN vectors comprise one or more modifications of the U3 region in the 3' LTR to prevent viral transcription beyond the first round of viral replication. This is because the right (3') LTR U3 region is used as a template for the left (5') LTR U3 region during viral replication and, thus, the viral transcript cannot be made without the U3 enhancer-promoter. In particular embodiments, the 3' LTR is modified such that the U3 region is deleted and the R and/or U5 region is replaced, for example, with a heterologous or synthetic poly(A) sequence, one or more insulator elements, and/or an inducible promoter.

One or more pDNAs can encode a transfer plasmid comprising a packageable viral vector genome and one or more of the viral structural/accessory proteins selected from the group consisting of: gag, pol, env, tat, rev, vif, vpr, vpu, vpx, and nef. The viral structural/accessory proteins are selected from the group consisting of: gag, pol, env, tat, and rev.

Viral envelope proteins (env) determine the range of host cells which can ultimately be infected and transformed by recombinant retroviruses generated from the cell lines. In the case of lentiviruses, such as HIV-1, HIV-2, SIV, FIV and EIV, the env proteins include gp41 and gp 120.

Illustrative examples of env genes include, but are not limited to: MLV envelopes, 10A1 envelope, BAEV, FeLV-B, RD114, SSAV, Ebola, Sendai, FPV (Fowl plague virus), and influenza virus envelopes. Similarly, genes encoding envelopes from RNA viruses (e.g., RNA virus families of Picornaviridae, Calciviridae, Astroviridae, Togaviridae, Flaviviridae, Coronaviridae, Paramyxoviridae, Rhabdoviridae, Filoviridae, Orthomyxoviridae, Bunyaviridae, Arenaviridae, Reoviridae, Birnaviridae, Retroviridae) as well as from the DNA viruses (families of Hepadnaviridae, Circoviridae, Parvoviridae, Papovaviridae, Adenoviridae, Herpesviridae, Poxyiridae, and Iridoviridae) may be utilized. Representative examples include, FeLV, VEE, HFVW, WDSV, SFV, Rabies, ALV, BIV, BLV, EBV, CAEV, SNV, ChTLV, STLV, MPMV, SMRV, RAV, FuSV, MH2, AEV, AMV, CT10, and EIAV.

Suitable env proteins include, but are not limited to any of the following viruses: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesviruses (HHV), human herpesvirus type 6 and 8, Human immunodeficiency virus (HIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), West Nile virus, any encephaliltis causing virus.

The env gene can encode a VSV-G envelope glycoprotein.

The pDNA/PEI complexes contemplated herein can comprise a transfer plasmid encoding a lentiviral vector comprising a modified left (5') lentiviral LTR comprising a heterologous promoter, a Psi packaging sequence (Ψ+), a cPPT/FLAP, an RRE, a promoter operably linked to a polynucleotide encoding a therapeutic transgene, and a modified SIN (3') lentiviral LTR; a plasmid encoding a lentiviral gag/pol, a plasmid encoding rev, and a plasmid encoding an env gene, preferably a VSV-G envelope glycoprotein.

The terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

The "subject" treated by the presently disclosed methods in their many embodiments is desirably a human subject, although it is to be understood that the methods described herein are effective with respect to all vertebrate species, which are intended to be included in the term "subject." Accordingly, a "subject" can include a human subject for medical purposes, such as for the treatment of an existing condition or disease or the prophylactic treatment for preventing the onset of a condition or disease, or an animal subject for medical, veterinary purposes, or developmental purposes. Suitable animal subjects include mammals including, but not limited to, primates, e.g., humans, monkeys, apes, and the like; bovines, e.g., cattle, oxen, and the like; ovines, e.g., sheep and the like; caprines, e.g., goats and the like; porcines, e.g., pigs, hogs, and the like; equines, e.g., horses, donkeys, zebras, and the like; felines, including wild and domestic cats; canines, including dogs; lagomorphs, including rabbits, hares, and the like; and rodents, including mice, rats, and the like. An animal may be a transgenic animal. In some embodiments, the subject is a human including, but not limited to, fetal, neonatal, infant, juvenile, and adult subjects. Further, a "subject" can include a patient afflicted with or suspected of being afflicted with a condition or disease. Thus, the terms "subject" and "patient" are used interchangeably herein. The term "subject" also refers to an organism, tissue, cell, or collection of cells from a subject.

In general, the "effective amount" of an active agent or drug delivery device refers to the amount necessary to elicit the desired biological response. As will be appreciated by those of ordinary skill in this art, the effective amount of an agent or device may vary depending on such factors as the desired biological endpoint, the agent to be delivered, the makeup of the pharmaceutical composition, the target tissue, and the like.

### EXAMPLES

The following Examples have been included to provide guidance to one of ordinary skill in the art for practicing representative embodiments of the presently disclosed subject matter.

### EXAMPLE 1

### 1.1 Overview

The presently disclosed subject matter provides a kinetically controlled approach to generate size-controlled and stable pDNA/lipid particles (200 nm - 1200 nm) using 60-nm LNPs building blocks. It has been identified that the average size of these particles is a determinant factor in achieving a high transfection efficiency for both *in vitro* and *in vivo.* For *in vitro* transfection, compared to 60-nm LNPs, the transfection efficiency of 1200-nm LNPs is 111-fold and 44-fold higher on HEK293T cells and HepG2 liver cancer cells, respectively. In addition, the size of the pDNA/lipid particles was identified as a key determinant for efficiency of *in vivo* local transfection via intrahepatic injection. The presently disclosed size-controlled LNPs will find a wide range of applications in both process engineering for production of viral vectors and gene therapy.

### 1.2 Representative Results

### 1.2.1 Production of stable pDNA/PEI particles with controlled size in the range of 40 to 1000 nm

In some embodiments, the presently disclosed subject matter provides a bottom-up assembly strategy based on the characteristics of kinetic growth of the pDNA/lipid particle system (FIG. 1A). First, uniform, small LNPs were prepared. These LNPs were stable and condensed due to hydrophobic effect. The uniform, small LNPs were subsequently used as building blocks for larger particles. Second, by adjusting the polarity of solution by adding a water-miscible organic solvent, for example, ethanol, methanol, butanol, isopropanol, DMSO, DMF, THF, acetone, acetonitrile, and the like, the kinetic stability of pDNA/lipid particle surface will be sufficiently removed. At this point, particle association and aggregation became possible and the particles grew gradually. This process can be effectively stopped when a desired particle size is reached, for example, by reversing the polarity of the solution via dilution with H₂O.

During the first step, uniform small pDNA/lipid nanoparticles were synthesized using a flash nanocomplexation (FNC) technique using a confined impinging jet (CIJ) mixer to induce turbulent mixing at high flow rates. The flash nanocomplexation technique is a kinetically controlled mixing process that can be used to accelerate the mixing of an anionic nucleic acid, e.g., pDNA, solution with a cationic lipid solution to match the PEC assembly kinetics through turbulent mixing in a microchamber, thus achieving explicit control of the kinetic conditions for pDNA/lipid nanoparticle assembly as demonstrated by the tunability of nanoparticle size, composition, and pDNA payload. See He et al., 2018, and International PCT Patent Application Publication No. WO2020223323 for Compositionally Defined Plasmid DNA/Polycation Nanoparticles and Methods for Making the Same, to Mao et al., published November 5, 2020, each of which is incorporated herein by reference in its entirety.

With an input pDNA concentration of 100 µg/mL, the designed nanoparticles had an average size of 60 nm measured by DLS (FIG. 1B). This approach was tested on a small batch scale with pipet mixing and vortexing. Challenging with a different volume of ethanol initiated a gradual size growth process for which the growth rate was determined by the final concentration of ethanol (FIG. 1C). By adjusting the concentration of ethanol, LNPs with a different ratio of positively chargeable polymer amine (N = nitrogen) groups to negatively charged nucleic acid phosphate (P) groups (N/P ratio) have a similar size growth kinetic profile (FIG. 1D). Proceeding with 40 to 70% ethanol (a dielectric constant of mixture from around 60 to 40) at room temperature and halting the growth by mixing the suspension with an equal volume of H₂O at different time points along its growth path successfully stabilized the average particle sizes at 200 nm, 300 nm, 400 nm, 500 nm, 600 nm, 800 nm and 1200 nm (FIG. 1E) with a high degree of consistency (FIG. 1F). These specific particle sizes were intentionally selected as a proof of principle; any other desired sizes can be readily achieved through halting the growth at different time points.

Based on the generic nature of this approach, pDNA/lipid particles with different compositions at different desired sizes can be achieved.

### 1.2.2 In vitro transfection efficiencies of stable pDNA/lipid particles with controlled size.

The stable particles were dosed to a monolayer culture of HEK293T cells and HepG2 cells to test the transfection efficiency using a pDNA encoding luciferase as a reporter. The dosing solution was diluted to achieve a working concentration of 1 µg pDNA/mL for transfection experiment. The luciferase activity readouts (FIG. 2A, FIG. 2B) verified, compared to 60-nm LNPs, the transfection efficiency of 1200-nm LNPs is 111-fold and 44-fold higher on HEK293T cells and HepG2 liver cancer cells, respectively.

### 1.2.3 In vivo local transfection efficiencies of stable pDNA/lipid particles with controlled size via intrahepatic injection

The stable particles with different sizes (100 nm, 600 nm and 1000 nm) were directly injected into liver to test the transfection efficiency using a pDNA encoding luciferase as a reporter (3 µg per mice) (FIG. 3A). As shown in FIG. 3B, the size of the pDNA/lipid particles also serves as a key determinant for efficiency for *in vivo* local transfection via intrahepatic injection. Compared to 100 nm or 1000 nm LNPs, the pDNA/lipid particles with a size of 600 nm yielded the highest transfection efficiency. The markedly elevated transgene level of 600-nm LNPs was further confirmed by luciferase activity readouts of single cell suspension of liver harvested after 48 h (FIG. 3C).

### REFERENCES

All publications, patent applications, patents, and other references mentioned in the specification are indicative of the level of those skilled in the art to which the presently disclosed subject matter pertains. All publications, patent applications, patents, and other references (e.g., websites, databases, etc.) mentioned in the specification are herein incorporated by reference in their entirety to the same extent as if each individual publication, patent application, patent, and other reference was specifically and individually indicated to be incorporated by reference. It will be understood that, although a number of patent applications, patents, and other references are referred to herein, such reference does not constitute an admission that any of these documents forms part of the common general knowledge in the art. In case of a conflict between the specification and any of the incorporated references, the specification (including any amendments thereof, which may be based on an incorporated reference), shall control. Standard art-accepted meanings of terms are used herein unless indicated otherwise. Standard abbreviations for various terms are used herein.

He, Z.; Hu, Y.; Nie, T.; Tang, H.; Zhu, J.; Chen, K.; Liu, L ; Leong, K. W.; Chen, Y.; Mao, H.-Q., Size controlled lipid nanoparticle production using turbulent mixing to enhance oral DNA delivery. Acta Biomaterialia 2018, 81, 195-207.

International PCT Patent Application Publication No. WO2020223323 for Compositionally Defined Plasmid DNA/Polycation Nanoparticles and Methods for Making the Same, to Mao et al., published November 5, 2020.

Although the foregoing subject matter has been described in some detail by way of illustration and example for purposes of clarity of understanding, it will be understood by those skilled in the art that certain changes and modifications can be practiced within the scope of the appended claims.

## Claims

1. A method for preparing a plurality of nucleic acid/lipid particles having an average particle size ranging from 210 nm to 1200 nm and a polydispersity index from 0.05 to 0.5, the method comprising:
(a) preparing or providing a first solution comprising a plurality of nucleic acid/lipid nanoparticles having a first particle size ranging from 20 to 200 nm;
(b) reducing a polarity of the first solution from a dielectric constant of about 80 to about 45 to 60 to induce particle-size growth of the plurality of nucleic acid/lipid nanoparticles having a first particle size to form a second solution comprising a plurality of nucleic acid/lipid nanoparticles having a second particle size, wherein the second particle size is in the range of 210 nm to 1200 nm;
(c) reversing the polarity of the second solution with a dielectric constant of about 65 to 80 to halt growth of the plurality of nucleic acid/lipid nanoparticles having a second particle size at a predetermined particle size, to form a plurality of nucleic acid/lipid particles having an average particle size ranging from 210 to 1200 nm and a polydispersity index from 0.05 to 0.5.

2. The method of claim 1, wherein the first particle size has a range between about 40 nm to about 120 nm, optionally wherein the first particle size is about 60 nm.

3. The method of claim 1 or 2, wherein the second particle size is selected from the group consisting of about 210, 300, 400, 500, 600, 700, 800, 900, 1000, 1100, and 1200 nm.

4. The method of any one of claims 1-3, wherein the first solution further comprises one or more multivalent cations selected from the group consisting of Ca²⁺, Mg²⁺, Zn²⁺, and Fe³⁺.

5. The method of any one of claims 1 to 4, wherein the polarity of the first solution is reduced by adding a water-miscible organic solvent thereto, optionally wherein the method comprises adding a varying concentration of the water-miscible organic solvent to the first solution to control the particle-size growth of the plurality of nucleic acid/lipid nanoparticles having a first particle size and/or optionally wherein the water-miscible organic solvent is selected from the group consisting of ethanol, methanol, butanol, isopropanol, dimethyl sulfoxide (DMSO), dimethylformamide (DMF), tetrahydrofuran (THF), acetone, and acetonitrile.

6. The method of any one of claims 1 to 5, wherein the polarity of the second solution is reversed via dilution with H₂O.

7. The method of any one of claims 1 to 6, wherein the first solution comprising a plurality of nucleic acid/lipid nanoparticles having a first particle size is prepared by:
(a) a continuous flash nanocomplexation (FNC) technique; or
(b) a manual pipette mixing and vortexing method.

8. The method of any one of claims 1 to 7, comprising an input nucleic acid concentration of about 100 µg/mL, optionally wherein the nucleic acid is selected from the group consisting of an antisense oligonucleotide, cDNA, genomic DNA, guide RNA, plasmid DNA, vector DNA, mRNA, miRNA, piRNA, shRNA, and siRNA, optionally wherein the nucleic acid comprises one or more different species of nucleic acids wherein optionally the nucleic acid comprises one or more plasmid DNAs.

9. The method of any one of claims 1-8, wherein the nucleic acid/lipid nanoparticle comprises one or more lipids selected from the group consisting of positively charged lipids, neutral lipids, negatively charged lipids, PEGylated lipids, and ionizable lipids.

10. The method of claim 9, wherein the nucleic acid/lipid nanoparticle comprises a cationic lipid, optionally wherein the cationic lipid is selected from the group consisting of N1-[2-((1S)-1-[(3-aminopropyl)amino]-4-[di(3-aminopropyl)amino]butylcarboxamido)ethyl]-3,4-di[oleyloxy]-benzamide, 1,2-di-O-octadecenyl-3-trimethylammonium propane (DOTMA), O-alkyl phosphatidylcholines, 1,2-dilauroyl-sn-glycero-3-ethylphosphocholine (12:0 EPD), 1,2-dimyristoyl-sn-glycero-3-ethylphosphocholine (14:0 EPC), 1,2-dipalmitoyl-sn-glycero-3-ethylphosphocholine (16:0 EPC), 1,2-distearoyl-sn-glycero-3-ethylphosphocholine (18:0 EPC), 1,2-dioleoyl-sn-glycero-3-ethylphosphocholine (18:1 EPC), 1-palmitoyl-2-oleoyl-sn-glycero-3-ethylphosphocholine (16:0-18:1 EPC), and 1,2-dimyristoleoyl-sn-glycero-3-ethylphosphocholine (14:1 EPC), dimethyldioctadecylammonium (DDAB), N-(4-carboxybenzyl)-N,N-dimethyl-2,3-bis(oleoyloxy)propan-1-aminium (DOBAQ), 1,2-distearoyl-3-dimethylammonium-propane (18:0 DAP), 1,2-dipalmitoyl-3-dimethylammonium-propane (16:0 DAP), 1,2-dimyristoyl-3-dimethylammonium-propane (14:0 DAP), 1,2-dioleoyl-3-dimethylammonium-propane (DODAP) (18:1 DAP), 1,2-dimyristoyl-3-trimethylammonium-propane (14:0 TAP), 1,2-dipalmitoyl-3-trimethylammonium-propane (16:0 TAP), 1,2-stearoyl-3-trimethylammonium-propane (18:0 TAP), 1,2-dioleoyl-3-trimethylammonium-propane (18:1 TAP (DOTAP)), dioleoylphosphatidylethanolamine (DOPE), 3β-[N-(N',N'-dimethylaminoethane)-carbamoyl]cholesterol hydrochloride (DC-Cholesterol·HCl), DC-cholesterol, N4-Cholesteryl-Spermine (GL67), 1,2-dioleyloxy-3-dimethylaminopropane (DODMA), dimyristoyltrimethylammonium propane (DMTAP), 2,3,-dioleyloxy-N-[2(sperminecarboxamido)ethyl]-N,N-dimethyl-1-propane trifluoroacetate (DOSPA), N,N-dioleyl-N,N-dimethylammonium chloride (DODAC), 1,2-Dioleoylcarbamyl-3-Dimethylammonium-propane (DOCDAP), 1,2-Dilineoyl-3-Dimethylammonium-propane (DLINDAP), dilauryl(C_{12:0}) trimethyl ammonium propane (DLTAP), dioctadecylamidoglycyl spermine (DOGS), DC-Choi, 1,2-Dimyristyloxypropyl-3-dimethyl-hydroxyethyl ammonium bromide (DMRIE), 3-dimethylamino-2-(Cholest-5-en-3-beta-oxybutan-4-oxy)-1-(cis,cis-9, 12-oc- tadecadienoxy)propane (CLinDMA), 2-[5'-(cholest-5-en-3[beta]-oxy)-3'-oxapentoxy)-3-dimethyl-1-(ci- s,cis-9',12'-octadecadienoxy) propane (CpLinDMA) and N,N-Dimethyl-3,4-dioleyloxybenzylamine (DMOBA), and 1,2-N,N'-Dioleylcarbamyl-3-dimethylaminopropane (DOcarbDAP), and combinations and pharmaceutically acceptable salts thereof.

11. The method of claim 10, wherein the cationic lipid is DOTAP.

## Patentansprüche

1. Ein Verfahren zur Herstellung einer Vielzahl von Nukleinsäure-/Lipidpartikeln mit einer durchschnittlichen Partikelgröße im Bereich von 210 nm bis 1200 nm und einem Polydispersitätsindex von 0,05 bis 0,5, wobei das Verfahren Folgendes beinhaltet:
(a) Herstellen oder Bereitstellen einer ersten Lösung, die eine Vielzahl von Nukleinsäure-/Lipidnanopartikeln mit einer ersten Partikelgröße im Bereich von 20 bis 200 nm beinhaltet;
(b) Reduzieren einer Polarität der ersten Lösung von einer Dielektrizitätskonstante von etwa 80 auf etwa 45 bis 60, um ein Partikelgrößenwachstum der Vielzahl von Nukleinsäure-/Lipidnanopartikeln mit einer ersten Partikelgröße zu induzieren, um eine zweite Lösung zu bilden, die eine Vielzahl von Nukleinsäure-/Lipidnanopartikeln mit einer zweiten Partikelgröße beinhaltet, wobei die zweite Partikelgröße im Bereich von 210 nm bis 1200 nm liegt;
(c) Umkehren der Polarität der zweiten Lösung mit einer Dielektrizitätskonstante von etwa 65 bis 80, um das Wachstum der Vielzahl von Nukleinsäure-/Lipidnanopartikeln mit einer zweiten Partikelgröße bei einer vorbestimmten Partikelgröße zu stoppen, um eine Vielzahl von Nukleinsäure-/Lipidpartikeln mit einer durchschnittlichen Partikelgröße im Bereich von 210 bis 1200 nm und einem Polydispersitätsindex von 0,05 bis 0,5 zu bilden.

2. Verfahren gemäß Anspruch 1, wobei die erste Partikelgröße einen Bereich zwischen etwa 40 nm bis etwa 120 nm aufweist, wobei die erste Partikelgröße optional etwa 60 nm beträgt.

3. Verfahren gemäß Anspruch 1 oder 2, wobei die zweite Partikelgröße aus der Gruppe bestehend aus etwa 210, 300, 400, 500, 600, 700, 800, 900, 1000, 1100 und 1200 nm ausgewählt ist.

4. Verfahren gemäß einem der Ansprüche 1-3, wobei die erste Lösung ferner ein oder mehrere mehrwertige Kationen beinhaltet, die aus der Gruppe bestehend aus Ca²⁺, Mg²⁺, Zn²⁺ und Fe³⁺ ausgewählt sind.

5. Verfahren gemäß einem der Ansprüche 1 bis 4, wobei die Polarität der ersten Lösung durch Zugabe eines wassermischbaren organischen Lösungsmittels dazu reduziert wird, wobei das Verfahren optional die Zugabe einer variierenden Konzentration des wassermischbaren organischen Lösungsmittels zur ersten Lösung beinhaltet, um das Partikelgrößenwachstum der Vielzahl von Nukleinsäure-/Lipidnanopartikeln mit einer ersten Partikelgröße zu steuern und/oder wobei das wassermischbare organische Lösungsmittel optional aus der Gruppe bestehend aus Ethanol, Methanol, Butanol, Isopropanol, Dimethylsulfoxid (DMSO), Dimethylformamid (DMF), Tetrahydrofuran (THF), Aceton und Acetonitril ausgewählt ist.

6. Verfahren gemäß einem der Ansprüche 1 bis 5, wobei die Polarität der zweiten Lösung durch Verdünnung mit H₂O umgekehrt wird.

7. Verfahren gemäß einem der Ansprüche 1 bis 6, wobei die erste Lösung, die eine Vielzahl von Nukleinsäure-/Lipidnanopartikeln mit einer ersten Partikelgröße beinhaltet, hergestellt wird durch:
(a) eine kontinuierliche Flash-Nanokomplexierungstechnik (FNC-Technik); oder
(b) ein Misch- und Verwirbelungsverfahren mittels manueller Pippette.

8. Verfahren gemäß einem der Ansprüche 1 bis 7, das eine Eingangsnukleinsäurekonzentration von etwa 100 µ/ml beinhaltet, wobei die Nukleinsäure optional aus der Gruppe bestehend aus einem Antisense-Oligonukleotid, cDNA, genomischer DNA, Führungs-RNA, Plasmid-DNA, Vektor-DNA, mRNA, miRNA, piRNA, shRNA und siRNA ausgewählt ist, wobei die Nukleinsäure optional eine oder mehrere verschiedene Arten von Nukleinsäuren beinhaltet, wobei die Nukleinsäure optional eine oder mehrere Plasmid-DNAs beinhaltet.

9. Verfahren gemäß einem der Ansprüche 1-8, wobei das Nukleinsäure-/Lipidnanopartikel ein oder mehrere Lipide beinhaltet, die aus der Gruppe bestehend aus positiv geladenen Lipiden, neutralen Lipiden, negativ geladenen Lipiden, PEGylierten Lipiden und ionisierbaren Lipiden ausgewählt sind.

10. Verfahren gemäß Anspruch 9, wobei das Nukleinsäure-/Lipidnanopartikel ein kationisches Lipid beinhaltet, wobei das kationische Lipid optional aus der Gruppe bestehend aus N1-[2-((1S)-1-[(3-Aminopropyl)amino]-4-[di(3-aminopropyl)amino]butylcarboxamido)ethyl]-3,4-di[oleyloxy]benzamid, 1,2-Di-O-octadecenyl-3-trimethylammoniumpropan (DOTMA), O-Alkylphosphatidylcholinen, 1,2-Dilauroyl-sn-glycero-3-ethylphosphocholin (12:0 EPD), 1,2-Dimyristoyl-sn-glycero-3-ethylphosphocholin (14:0 EPC), 1,2-Dipalmitoyl-sn-glycero-3-ethylphosphocholin (16:0 EPC), 1,2-Distearoyl-sn-glycero-3-ethylphosphocholin (18:0 EPC), 1,2-Dioleoyl-sn-glycero-3-ethylphosphocholin (18:1 EPC), 1-Palmitoyl-2-oleoyl-sn-glycero-3-ethylphosphocholin (16:0-18:1 EPC) und 1,2-Dimyristoleoyl-sn-glycero-3-ethylphosphocholin (14:1 EPC), Dimethyldioctadecylammonium (DDAB), N-(4-Carboxybenzyl)-N,N-dimethyl-2,3-bis(oleoyloxy)propan-1-aminium (DOBAQ), 1,2-Distearoyl-3-dimethylammoniumpropan (18:0 DAP), 1,2-Dipalmitoyl-3-dimethylammoniumpropan (16:0 DAP), 1,2-Dimyristoyl-3-dimethylammoniumpropan (14:0 DAP), 1,2-Dioleoyl-1-3-dimethylammoniumpropan (DODAP) (18:1 DAP), 1,2-Dimyristoyl-3-trimethylammoniumpropan (14:0 TAP), 1,2-Dipalmitoyl-3-trimethylammoniumpropan (16:0 TAP), 1,2-Stearoyl-1-3-trimethylammoniumpropan (18:0 TAP), 1,2-Dioleoyl-3-trimethylammoniumpropan (18:1 TAP (DOTAP)), Dioleoylphosphatidylethanolamin (DOPE), 3β-[N-(N',N'-Dimethylaminoethan)carbamoyl]cholesterolhydrochlorid (DC-Cholesterol·HCl), DC-Cholesterol, N4-Cholesterylspermin (GL67), 1,2-Dioleyloxy-3-dimethylaminopropan (DODMA), Dimyristoyltrimethylammoniumpropan (DMTAP), 2,3-Dioleyloxy-N-[2(spermincarboxamido)ethyl]-N,N-dimethyl-1-propantrifluoracetat (DOSPA), N,N-Dioleyl-N,N-dimethylammoniumchlorid (DODAC), 1,2-Dioleoylcarbamyl-1-3-dimethylammoniumpropan (DOCDAP), 1,2-Dilineoy-1-3-dimethylammoniumpropan (DLINDAP), Dilauryl(C_{12:0})trimethylammoniumpropan (DLTAP), Dioctadecylamidoglycylspermin (DOGS), DC-Choi, 1,2-Dimyristyloxypropyl-3-dimethylhydroxyethylammoniumbromid (DMRIE), 3-Dimethylamino-2-(cholest-5-en-3-beta-oxybutan-4-oxy)-1-(cis,cis-9,12-oc-tadecadienoxy)propan (CLinDMA), 2-[5'-(cholest-5-en-3[beta]-oxy)-3'-oxapentoxy)-3-dimethyl-1-(cis-s,cis-9', 12'-octadecadienoxy)propan (CpLinDMA) und N,N-Dimethyl-3,4-dioleyloxybenzylamin (DMOBA) und 1,2-N,N'-Diolylcarbamyl-3-dimethylaminopropan (DOcarbDAP) und Kombinationen und pharmazeutisch verträglichen Salzen davon ausgewählt ist.

11. Verfahren gemäß Anspruch 10, wobei das kationische Lipid DOTAP ist.

## Revendications

1. Un procédé pour la préparation d'une pluralité de particules d'acide nucléique/lipide ayant une taille moyenne de particule comprise dans la plage allant de 210 nm à 1 200 nm et un indice de polydispersité allant de 0,05 à 0,5, le procédé comprenant :
(a) la préparation ou la fourniture d'une première solution comprenant une pluralité de nanoparticules d'acide nucléique/lipide ayant une première taille de particule comprise dans une plage allant de 20 à 200 nm ;
(b) la réduction d'une polarité de la première solution d'une constante diélectrique d'environ 80 à environ 45 à 60 pour induire une croissance de taille de particule de la pluralité de nanoparticules d'acide nucléique/lipide ayant une première taille de particule afin de former une deuxième solution comprenant une pluralité de nanoparticules d'acide nucléique/lipide ayant une deuxième taille de particule, où la deuxième taille de particule est comprise dans la plage allant de 210 nm à 1 200 nm ;
(c) l'inversion de la polarité de la deuxième solution avec une constante diélectrique d'environ 65 à 80 pour arrêter la croissance de la pluralité de nanoparticules d'acide nucléique/lipide ayant une deuxième taille de particule à une taille de particule prédéterminée, afin de former une pluralité de particules d'acide nucléique/lipide ayant une taille moyenne de particule comprise dans la plage allant de 210 à 1 200 nm et un indice de polydispersité allant de 0,05 à 0,5.

2. Le procédé de la revendication 1, où la première taille de particule a une plage comprise entre environ 40 nm et environ 120 nm, facultativement où la première taille de particule est d'environ 60 nm.

3. Le procédé de la revendication 1 ou 2, où la deuxième taille de particule est sélectionnée dans le groupe constitué d'environ 210, 300, 400, 500, 600, 700, 800, 900, 1 000, 1 100, et 1 200 nm.

4. Le procédé de l'une quelconque des revendications 1 à 3, où la première solution comprend en outre un ou plusieurs cations multivalents sélectionnés dans le groupe constitué de Ca²⁺, Mg²⁺, Zn²⁺, et Fe³⁺.

5. Le procédé de l'une quelconque des revendications 1 à 4, où la polarité de la première solution est réduite par l'ajout d'un solvant organique miscible à l'eau dans celle-ci, facultativement où le procédé comprend l'ajout d'une concentration variable du solvant organique miscible à l'eau dans la première solution pour contrôler la croissance de taille de particule de la pluralité de nanoparticules d'acide nucléique/lipide ayant une première taille de particule et/ou facultativement où le solvant organique miscible à l'eau est sélectionné dans le groupe constitué d'éthanol, méthanol, butanol, isopropanol, diméthylsulfoxyde (DMSO), diméthylformamide (DMF), tétrahydrofurane (THF), acétone, et acétonitrile.

6. Le procédé de l'une quelconque des revendications 1 à 5, où la polarité de la deuxième solution est inversée par dilution avec H₂O.

7. Le procédé de l'une quelconque des revendications 1 à 6, où la première solution comprenant une pluralité de nanoparticules d'acide nucléique/lipide ayant une première taille de particule est préparée par :
(a) une technique de nanocomplexation flash continue (FNC) ; ou
(b) un procédé de mélange et de vortexage par pipette à la main.

8. Le procédé de l'une quelconque des revendications 1 à 7, comprenant une concentration d'acide nucléique d'entrée d'environ 100 µg/mL, facultativement où l'acide nucléique est sélectionné dans le groupe constitué d'un oligonucléotide antisens, ADNc, ADN génomique, ARN guide, ADN plasmidique, ADN vecteur, ARNm, ARNmi, ARNpi, ARNsh, et ARNsi, facultativement où l'acide nucléique comprend une ou plusieurs espèces différentes d'acides nucléiques où facultativement l'acide nucléique comprend un ou plusieurs ADN plasmidiques.

9. Le procédé de l'une quelconque des revendications 1 à 8, où la nanoparticule d'acide nucléique/lipide comprend un ou plusieurs lipides sélectionnés dans le groupe constitué de lipides chargés positivement, lipides neutres, lipides chargés négativement, lipides pégylés, et lipides ionisables.

10. Le procédé de la revendication 9, où la nanoparticule d'acide nucléique/lipide comprend un lipide cationique, facultativement où le lipide cationique est sélectionné dans le groupe constitué de N1-[2-((1S)-1-[(3-aminopropyl)amino]-4-[di(3-aminopropyl)amino]butylcarboxamido)éthyl]-3,4-di[oléyloxy]-benzamide, 1,2-di-O-octadécényl-3-triméthylammonium propane (DOTMA), O-alkylphosphatidylcholines, 1,2-dilauroyl-sn-glycéro-3-éthylphosphocholine (12:0 EPD), 1,2-dimyristoyl-sn-glycéro-3-éthylphosphocholine (14:0 EPC), 1,2-dipalmitoyl-sn-glycéro-3-éthylphosphocholine (16:0 EPC), 1,2-distéaroyl-sn-glycéro-3-éthylphosphocholine (18:0 EPC), 1,2-dioléoyl-sn-glycéro-3-éthylphosphocholine (18:1 EPC), 1-palmitoyl-2-oléoyl-sn-glycéro-3-éthylphosphocholine (16:0-18:1 EPC), et 1,2-dimyristoléoyl-sn-glycéro-3-éthylphosphocholine (14:1 EPC), diméthyldioctadécylammonium (DDAB), N-(4-carboxybenzyl)-N,N-diméthyl-2,3-bis(oléoyloxy)propan-1-aminium (DOBAQ), 1,2-distéaroyl-3-diméthylammonium-propane (18:0 DAP), 1,2-dipalmitoyl-3-diméthylammonium-propane (16:0 DAP), 1,2-dimyristoyl-3-diméthylammonium-propane (14:0 DAP), 1,2-dioléoyl-3-diméthylammonium-propane (DODAP) (18:1 DAP), 1,2-dimyristoyl-3-triméthylammonium-propane (14:0 TAP), 1,2-dipalmitoyl-3-triméthylammonium-propane (16:0 TAP), 1,2-stéaroyl-3-triméthylammonium-propane (18:0 TAP), 1,2-dioléoyl-3-triméthylammonium-propane (18:1 TAP (DOTAP)), dioléoylphosphatidyléthanolamine (DOPE), chlorhydrate de 3β-[N-(N',N'-diméthylaminoéthane)-carbamoyl]cholestérol (DC-Cholestérol·HCl), DC-cholestérol, N4-cholestéryl-spermine (GL67), 1,2-dioléyloxy-3-diméthylaminopropane (DODMA), dimyristoyltriméthylammonium propane (DMTAP), trifluoroacétate de 2,3-dioléyloxy-N-[2(sperminecarboxamido)éthyl]-N,N-diméthyl-1-propane (DOSPA), chlorure de N,N-dioléyl-N,N-diméthylammonium (DODAC), 1,2-dioléoylcarbamyl-3-diméthylammonium-propane (DOCDAP), 1,2-dilinéoyl-3-diméthylammonium-propane (DLINDAP), dilauryl(C_{12:0}) triméthylammonium propane (DLTAP), dioctadécylamidoglycyl spermine (DOGS), DC-Choi, bromure de 1,2-dimyristyloxypropyl-3-diméthyl-hydroxyéthylammonium (DMRIE), 3-diméthylamino-2-(cholest-5-én-3-bêta-oxybutan-4-oxy)-1-(cis,cis-9,12-octadécadiénoxy)propane (CLinDMA), 2-[5'-(cholest-5-én-3[bêta]-oxy)-3'-oxapentoxy)-3-diméthyl-1-(cis,cis-9',12'-octadécadiénoxy)propane (CpLinDMA) et N,N-diméthyl-3,4-dioléyloxybenzylamine (DMOBA), et 1,2-N,N'-dioléylcarbamyl-3-diméthylaminopropane (DOcarbDAP), et des combinaisons et des sels pharmaceutiquement acceptables de ceux-ci.

11. Le procédé de la revendication 10, où le lipide cationique est DOTAP.
